# EUROPÄISCHE PATENTSCHRIFT

(11) **EP 4 051 143 B1**
(45) Veröffentlichungstag und Bekanntmachung des Hinweises auf die Patenterteilung: **28.05.2025**
(21) Anmeldenummer: 20800810.2
(22) Anmeldetag: 26.10.2020
(51) Int. Cl.: A61B 17/70

(54) **MEDIZINTECHNISCHES ENTKOPPLUNGSINSTRUMENT**
MEDICAL DECOUPLING DEVICE
INSTRUMENT À USAGE MÉDICAL DE DÉCROCHAGE

(30) Priorität: 28.10.2019 DE 102019129037
(43) Veröffentlichungstag der Anmeldung: 07.09.2022
(73) Patentinhaber: Aesculap AG, 78532 Tuttlingen (DE)
(72) Erfinder: ZOING TAN, Jian, 78532 Tuttlingen (DE); GNOTH, Barbara, 78194 Immendingen (DE); RICCI, Denis, 78573 Wurmlingen (DE)
(74) Vertreter: Winter, Brandl - Partnerschaft mbB
(86) Internationale Anmeldenummer: PCT/EP2020/080073
(87) Internationale Veröffentlichungsnummer: WO 2021/083845

(56) Entgegenhaltungen:
- US-A1- 2009 234 395
- US-A1- 2014 222 081
- US-A1- 2014 371 756

## Beschreibung

Die vorliegende Erfindung betrifft ein medizintechnisches Entkopplungsinstrument zum Entkoppeln eines medizintechnischen Einsetzinstruments von einer Pedikelschraube.

### Stand der Technik

Pedikelschrauben dienen prinzipiell zur dorsalen Stabilisierung der Wirbelsäule bei Frakturen, Tumoren, Entzündungen, Deformitäten und degenerativ bedingten Instabilitäten mittels transpedikulärer Verschraubung. Dabei werden Pedikelschrauben in den Pedikeln jeweils benachbarter Wirbel platziert, worauf eine winkelstabile Verbindung zwischen den jeweils axial übereinander sich anordnenden Pedikelschrauben und einem axial sich erstreckenden Längsträger/ Stab geschaffen wird. Die Pedikelschrauben und Längsträger bilden dabei ein Wirbel-Stabilisierungssystem.

Hierfür hat eine Pedikelschraube in der Regel einen axialen, schaftartigen Außengewindeabschnitt, an den sich schraubenkopfseitig eine sogenannte Tulpe bzw. Aufnahmehülse anschließt. Diese bildet konstruktiv eine U-förmig geschlitzte/ getunnelte Aufnahmehülse mit Innengewinde, wobei die beiden sich radial gegenüberliegenden Längsschlitze jeweils einen Schlitzspalt vorbestimmter Spaltbreite definieren. In die parallel zueinander verlaufenden Längsschlitze ist der Längsträger/ Stab quer eingelegt, der mittels eines Verriegelungselements, zum Beispiel in Form einer Madenschraube, Gewindemutter oder Setscrew, die in das Innengewinde eingedreht ist, fixiert wird.

Grundsätzlich werden zwei Grundarten von Pedikelschrauben unterschieden, nämlich monoaxiale sowie polyaxiale Pedikelschrauben. Im Fall einer monoaxialen Pedikelschraube sind der Außengewindeabschnitt/ Schaft und die Tulpe/ Aufnahmehülse einstückig miteinander ausgebildet. Eine polyaxiale Pedikelschraube hat hingegen einen als separates Bauteil gefertigten Außengewindeschaft mit einem zumeist kugelförmigen oder (semi-) sphärischen Schraubenkopf, der von der Aufnahmehülse/ Tulpe relativ verschwenkbar umgriffen und gleichzeitig im Übergangsbereich zwischen Kopf und Schaft hintergriffen wird. Auf diese Weise kann die Aufnahmehülse/ Tulpe nach Versenken des Außengewindeschafts im Pedikelkanal eines Wirbels relativ dazu verschwenkt und/oder verdreht werden, um eine gewünschte Lage und Ausrichtung im Wesentlichen unabhängig zur Ausrichtung des Schafts zu erhalten. Die Hinterschneidung verhindert dabei, dass die Aufnahmehülse/ Tulpe vom Schaftkopf abgezogen werden kann.

Pedikelschrauben werden von einem Operateur in den Pedikelkanal eines Wirbels eingesetzt bzw. durch Verschrauben verankert. Dabei bedient sich der Operateur unter anderem eines sogenannten Einsetzinstruments, welches auch als "Downtube" bezeichnet wird. Das Einsetzinstrument kann an seinem distalen Ende beispielsweise zwei Koppelarme aufweisen, an deren radial innerer Seite Raststrukturen, etwa Rastnasen, ausgebildet sind, die mit entsprechenden Gegenraststrukturen, welche an der Aufnahmehülse/ Tulpe der Pedikelschraube ausgebildet sind, in Eingriff gebracht werden können.

Möchte der Operateur nach erfolgter Verschraubung/ Verankerung der Pedikelschraube das Einsetzinstrument von der Pedikelschraube entkoppeln, ist ein Entkopplungsinstrument notwendig, welches auch als "Removal Key" bezeichnet wird. Das Entkopplungsinstrument wird dabei grundsätzlich in das Einsetzinstrument eingeführt und spreizt das Einsetzinstrument, insbesondere die Koppelarme des Einsetzinstruments (definierte elastische Deformation), wodurch die Entkopplung realisiert werden kann.

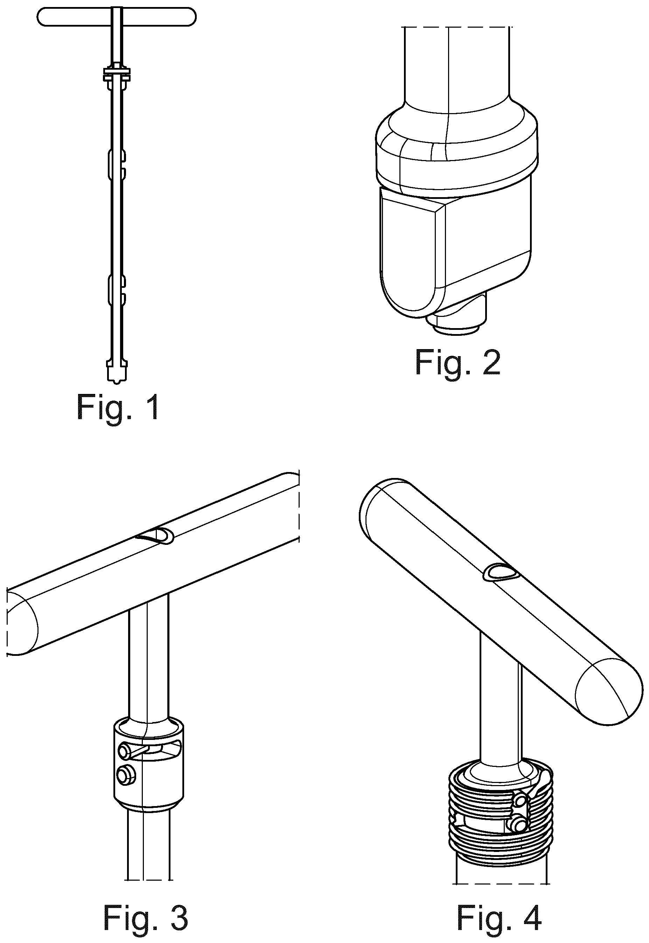

Aus dem Stand der Technik (siehe Fig. 1 bis Fig. 4) ist beispielsweise ein Entkopplungsinstrument bekannt, welches in ein Einsetzinstrument eingeführt werden kann. Fig. 4 zeigt beispielsweise einen oberen Abschnitt des Einsetzinstruments, in welches das Entkopplungsinstrument eingesetzt ist.

Das aus dem Stand der Technik bekannte Entkopplungsinstrument (siehe insbesondere Fig. 1 bis 3) weist grundsätzlich ein hohlzylinderartiges/ hülsenartiges Bauteil mit proximalem Griffabschnitt auf. In dem hohlzylinderartigen/ hülsenartigen Bauteil ist ein zylindrischer Stab aufgenommen, welcher an seinem distalen Ende ein Eingriffselement aufweist, das formschlüssig mit einer Pedikelschraube in Eingriff gelangen soll (siehe insbesondere unteren Abschnitt in Fig. 2). An dem proximalen Ende des zylindrischen Stabs ist ein Eingriffszapfen angebunden/ befestigt, welcher in einer in dem hohlzylinderartigen/ hülsenartigen Bauteil vorgesehenen Kulisse (Langloch) aufgenommen ist. Wenn der zylindrische Stab formschlüssig mit der Pedikelschraube in Eingriff ist, erlaubt der Eingriffszapfen eine Drehung des hohlzylinderartigen/ hülsenartigen Bauteils um etwa 90°. Die Kulisse bzw. das Langloch erstreckt sich in Umfangsrichtung des hohlzylinderartigen/ hülsenartigen Bauteils. An dem distalen Ende des hohlzylinderartigen/ hülsenartigen Bauteils ist ein ovaler Eingriffsabschnitt vorgesehen (siehe insbesondere oberen Abschnitt in Fig. 2). Wird das hohlzylinderartige/ hülsenartige Bauteil ausgehend von seiner Ausgangsposition/ Nullposition um etwa 90° verdreht, kann der ovale Eingriffsabschnitt des Entkopplungsinstruments das Einsetzinstrument entkoppeln, insbesondere Koppelarme des Einsetzinstruments spreizen. Das Entkopplungsinstrument ist grundsätzlich sowohl für die Ankopplung als auch für die Entkopplung von Pedikelschrauben verwendbar.

Der Stand der Technik, welcher aus den Fig. 1 bis 4 hervorgeht, hat grundsätzlich den Nachteil, dass vor einem Einführen des Entkopplungsinstruments in das Einsetzinstrument das hohlzylinderartige/ hülsenartige Bauteil und der zylindrische Stab zueinander manuell ausgerichtet werden müssen. Weiterhin kann der Formschluss am distalen Ende zwischen der Pedikelschraube und dem Entkopplungsinstrument die Entkopplung erschweren.

Aus der US 2014/371756 A1 ist ein medizinisches Einsetzinstrument zum Einsetzen einer Pedikelschraube mit integrierter Entkopplungsfunktionalität bekannt. Das Einsetzinstrument hat am distalen Ende eines Schafts ein Eingriffsende, welches mit einer in einer Pedikelschraube vorgesehenen Ausnehmung in Eingriff gebracht werden kann. Proximal des Eingriffsendes weist das Einsetzinstrument ein Eingriffselement mit flexiblen Federelementen auf, die mit der Tulpe der Pedikelschraube in Eingriff bringbar sind. Zwischen einer proximalen Endfläche des Eingriffselements und einer Hülse ist eine Feder vorgesehen. Die Hülse kann gegen eine Schulter der Pedikelschraube gedrückt werden. In diesem Zustand kann das Einsetzinstrument von der Pedikelschraube entkoppelt bzw. herausgezogen werden.

Die US 2009/234395 A1 offenbart ein medizinisches Instrument, in welchem bei einer Betätigung eines Hebels ein Pin in einer schrägen bzw. gekrümmten Kulisse nach vorne wandert, wodurch eine Drehbewegung von Betätigungselementen bewirkt wird. Dabei kann eine Pedikelschraube fest gehalten werden.

Die US 2014/222081 A1 offenbart einen Retraktor und einen Reducer, wobei der Reducer eine spiralförmig verlaufende Kulisse aufweist. Über eine Drehbewegung des Reducers wird einer Pedikelschraube ein Stab zugeführt.

### Kurzbeschreibung der Erfindung

Aufgabe der vorliegenden Erfindung ist es vor diesem Hintergrund, die Nachteile aus dem Stand der Technik zu vermeiden oder wenigstens zu mildern. Insbesondere soll ein medizintechnisches Entkopplungsinstrument zum Entkoppeln eines medizintechnischen Einsetzinstruments von einer Pedikelschraube bereitgestellt werden, welches in einfacher Weise an das medizintechnische Einsetzinstrument ankoppelbar ist und eine sichere Entkopplung bereitstellt (insbesondere das medizintechnische Einsetzinstrument definiert elastisch verformt).

Diese Aufgabe wird durch ein medizintechnisches Entkopplungsinstrument nach Anspruch 1 und eine medizintechnische Anordnung nach Anspruch 7 gelöst. Vorteilhafte Ausführungsformen und Weiterbildungen sind in den Unteransprüchen beansprucht und/oder werden nachfolgend erläutert. Im Nachfolgenden werden die Begriffe "proximal" und "distal" derart verwendet, dass "proximal" näher an dem Anwender/ Operateur und "distal" weiter von dem Anwender/ Operateur entfernt bedeutet.

Die Erfindung betrifft zunächst ein medizintechnisches Entkopplungsinstrument zum Entkoppeln eines medizintechnischen Einsetzinstruments von einer Pedikelschraube, insbesondere durch Aufspreizen des medizintechnischen Einsetzinstruments, mit: einem Schaft/ Stab und einer um einen (insbesondere zylindrischen) Abschnitt des Schafts/ Stabs angeordneten Hülse, welche zur definierten, insbesondere verdrehsicheren, Ankopplung an das medizintechnische Einsetzinstrument eingerichtet ist, wobei der Schaft/ Stab (zum Entkoppeln des medizintechnischen Einsetzinstruments von der Pedikelschraube) bezüglich der Hülse verdrehbar ist, und wobei das medizintechnische Entkopplungsinstrument eingerichtet ist, den Schaft/ Stab und die Hülse selbsttätig und automatisch in eine definierte Ausgangsposition/ Nullposition zu verbringen.

Das erfindungsgemäße medizintechnische Entkopplungsinstrument stellt somit eine definierte Nullposition/ Ausgangsposition bereit und es ist bevorzugt kein manuelles Ausrichten des Schafts und der Hülse vonnöten. In anderen Worten sind der Schaft und die Hülse des erfindungsgemäßen medizintechnischen Entkopplungsinstruments automatisch zueinander für das Einsetzen/ Einführen in das medizintechnische Einsetzinstrument ausgerichtet.

Das medizintechnisches Entkopplungsinstrument weist ferner eine Feder, insbesondere Spiralfeder/ Druckfeder, auf, welche zwischen einer an dem Schaft vorgesehenen Auflagefläche und der Hülse angeordnet ist und eingerichtet ist, über ihre Federkraft den Schaft und die Hülse in die definierte Ausgangsposition/ Nullposition zu verbringen. Insbesondere weist dabei die Hülse an ihrem distalen Ende eine Anlagefläche für die Feder auf.

Der Schaft weist an dem Abschnitt, um welchen die Hülse angeordnet ist, einen pinförmigen/ zapfenförmigen Vorsprung auf, die Hülse weist eine Kulisse/ ein Langloch auf, und der zapfenförmige Vorsprung des Schafts ist in der Kulisse der Hülse aufgenommen/ angeordnet, und zwar derart, dass die Kulisse einen Verdrehbereich/ eine Verdrehbarkeit (Winkelbereich) des Schafts in Bezug auf die Hülse definiert.

Die Kulisse verläuft schräg, erstreckt sich also sowohl in Umfangsrichtung als auch in Axialrichtung der Hülse, so dass ein erstes Ende der Kulisse näher an einem distalen Ende der Hülse angeordnet ist als ein zweites Ende der Kulisse.

Von besonderem Vorteil ist es, wenn in der definierten Ausgangsposition der zapfenförmige Vorsprung des Schafts gegen das erste Ende der Kulisse durch die Feder gedrückt ist. In anderen Worten bringt die Feder bevorzugt eine Druckkraft auf die Hülse auf, und zwar derart, dass sich der zapfenförmige Vorsprung/ Pin/ Zapfen des Schafts in der ursprünglichen Position/ Ausgangsposition/ Nullposition an dem ersten Ende der Kulisse befindet.

In vorteilhafter Weise erlaubt die Kulisse der Hülse etwa/ näherungsweise eine 90°-Drehung des Schafts. In anderen Worten erstreckt sich das Langloch/ die Kulisse von dem ersten Ende zu dem zweiten Ende in der Umfangsrichtung um etwa/ näherungsweise 90°.

Ein bevorzugtes Ausführungsbeispiel ist dadurch gekennzeichnet, dass die Kulisse an ihrem zweiten Ende eine Vertiefung/ Einkerbung/ Mulde (in Axialrichtung hin zu dem distalen Ende der Hülse) aufweist, so dass der zapfenförmige Vorsprung des Stabs über die Feder in die Vertiefung gedrückt werden kann. Dadurch wird ermöglicht, dass einem Anwender/ Operateur ein taktiles Feedback gegeben wird, wenn das medizintechnische Einsetzinstrument von der Pedikelschraube entkoppelt ist.

Es ist zweckmäßig, wenn die Hülse zur definierten Ankopplung an das medizintechnische Einsetzinstrument pinförmige/ zapfenförmige Vorsprünge aufweist, welche sich insbesondere diametral gegenüberliegen und radial nach außen erstrecken.

Dabei sind die zapfenförmigen Vorsprünge bevorzugt eingerichtet, dass sie mit dem medizintechnischen Einsetzinstrument derart in Eingriff gebracht werden können, dass die Hülse in Umfangsrichtung verdrehsicher in dem medizintechnischen Einsetzinstrument gehalten ist/ wird.

Außerdem ist es von Vorteil, wenn der Schaft einen proximalen Eingriffsabschnitt aufweist, welcher zylindrisch mit unrundem Querschnitt bzw. ovalzylindrisch geformt ist, und insbesondere eingerichtet ist, mit dem medizinischen Einsetzinstrument für eine Drehmomentübertragung in Eingriff gebracht zu werden/ dieses aufzuspreizen.

Bevorzugt weist ein Abschnitt des Schafts ein Druckstück auf, welches eingerichtet ist, eine Druckkraft radial nach außen zu erzeugen/ aufzubringen, insbesondere gegen das medizintechnische Einsetzinstrument zu drücken. Insbesondere ist das Druckstück derart ausgebildet bzw. eingerichtet, dass es eine Druckkraft radial nach außen gegen das medizintechnische Einsetzinstrument ausüben kann, damit ein unbeabsichtigtes Herausrutschen des medizintechnischen Entkopplungsinstruments verhindert wird.

In vorteilhafter Weise weist der Schaft einen proximalen Griffabschnitt auf, welcher im Wesentlichen zylinderförmig mit einem runden/ kreisförmigen oder unrunden Querschnitt geformt ist. Bevorzugt ist der proximale Griffabschnitt geriffelt bzw. weist eine Vielzahl von sich in Axialrichtung erstreckende längliche Ausnehmungen/ Vertiefungen auf, welche insbesondere in Umfangsrichtung gleichmäßig beabstandet/ verteilt sind, für ein verbessertes Greifen (und Verdrehen) des Schafts durch einen Anwender/ Operateur.

Bevorzugt schließt sich an den proximalen Griffabschnitt, welcher an einem proximalen Ende des Schafts vorgesehen ist, in Axialrichtung ein erster zylindrischer Abschnitt an. Der erste zylindrische Abschnitt des Schafts ist insbesondere der Abschnitt des Schafts, um welchen die, hohlzylinderförmig ausgebildete, Hülse angeordnet ist, und somit der Abschnitt des Schafts, welcher den sich radial nach außen erstreckenden zapfenförmigen/ zylindrischen Vorsprung aufweist. Der erste zylindrische Abschnitt des Schafts weist bevorzugt einen runden/ kreisförmigen Querschnitt auf. Der an dem ersten zylinderförmigen Abschnitt angeordnete zapfenförmige Vorsprung des Schafts ist bevorzugt in der Kulisse der Hülse aufgenommen.

Es ist von Vorteil, wenn sich an den ersten zylindrischen Abschnitt des Schafts in Axialrichtung ein zweiter zylindrischer Abschnitt des Schafts anschließt. Der zweite zylindrische Abschnitt hat insbesondere einen runden/ kreisförmigen Querschnitt. Bevorzugt ist ein Durchmesser des zweiten zylindrischen Abschnitts größer als ein Durchmesser des ersten zylindrischen Abschnitts. An einem proximalen Ende des zweiten zylindrischen Abschnitts ist in vorteilhafter Weise die Auflagefläche für die Feder vorgesehen/ ausgebildet. Das Druckstück ist bevorzugt an dem zweiten zylindrischen Abschnitt des Schafts vorgesehen.

Es ist zweckmäßig, wenn sich an den zweiten zylindrischen Abschnitt in Axialrichtung ein dritter zylindrischer Abschnitt anschließt, welcher insbesondere einen runden/ kreisförmigen Querschnitt aufweist und dessen Durchmesser bevorzugt kleiner als der Durchmesser des zweiten zylindrischen Abschnitts ist. Dabei muss dieser Abschnitt nicht zwangsläufig rund sein, solange der Durchmesser/die größte Breite an keiner Stelle größer ist als der Durchmesser des zweiten zylindrischen Abschnitts.

In vorteilhafter Weise schließt sich an den dritten zylindrischen Abschnitt in Axialrichtung der distale Eingriffsabschnitt an, welcher an einem distalen Ende des Schafts vorgesehen/ angeordnet ist.

Außerdem betrifft die Erfindung eine medizintechnische Anordnung umfassend zumindest das voranstehend beschriebene medizintechnische Entkopplungsinstrument und ein medizintechnisches Einsetzinstrument.

Das medizintechnische Entkopplungsinstrument ist bevorzugt eingerichtet, das medizintechnische Einsetzinstrument von der Pedikelschraube zu entkoppeln.

In vorteilhafter Weise wird die Entkopplung realisiert, indem das medizintechnische Entkopplungsinstrument eingerichtet ist, das medizintechnische Einsetzinstrument zu entkoppeln, insbesondere zwei einander diametral gegenüberliegende, sich parallel in Axialrichtung erstreckende Koppelarme des Einsetzinstruments von einer Pedikelschraube.

Bevorzugt weisen die Koppelarme des Einsetzinstruments an ihren distalen Enden jeweils eine Raststruktur auf, welche mit einer Gegenraststruktur in Eingriff bringbar ist, welche an einer Aufnahmehülse/ Tulpe einer polyaxialen oder monoaxialen Pedikelschraube ausgebildet ist.

Es ist zweckmäßig, wenn der unrunde, distale Eingriffsabschnitt des medizintechnischen Entkopplungsinstruments eingerichtet ist, durch Verdrehen des Schafts mit den Koppelarmen des medizintechnischen Einsetzinstruments in Eingriff gebracht zu werden, insbesondere die Koppelarme von der Pedikelschraube zu entkoppeln.

Es ist ferner von Vorteil, wenn die zapfenförmigen Vorsprünge der Hülse verdrehsicher in dem medizintechnischen Einsetzinstrument aufgenommen sind. Dazu weist das medizintechnische Einsetzinstrument bevorzugt an seinem proximalen Ende zwei einander diametral gegenüberliegende, sich in Axialrichtung erstreckende V-förmige bzw. U-förmige Ausnehmungen auf, welche die zapfenförmigen Vorsprünge der Hülse aufnehmen und verdrehsicher halten.

Mit anderen Worten betrifft die Erfindung ein (medizintechnisches) Entkopplungsinstrument ("Removal Key"), welches einen Schaft, eine Hülse und einen (Druck-)Federmechanismus enthält.

Der Schaft weist einen Pin/ Zapfen auf, die Hülse weist eine Kulisse/ ein Langloch auf und der Pin/ Zapfen des Schafts ist in der Kulisse/ dem Langloch der Hülse derart aufgenommen, dass Hülse und Schaft zueinander verdreht werden können.

Der Schaft hat an seinem distalen Ende eine unrunde Form, mittels welcher ein (medizintechnisches) Einsetzinstrument ("Downtube") zum Entkoppeln des Einsetzinstruments von einer Pedikelschraube gespreizt werden kann.

Außerdem enthält der Schaft ein Druckstück bzw. einen Einrastmechanismus, welches/r das Entkopplungsinstrument in dem Einsetzinstrument hält.

Der Federmechanismus ist derart zwischen dem Schaft und der Hülse angeordnet, dass er sicherstellt, dass sich die Hülse immer/ von selbst/ selbsttätig/ automatisch in einer Nullposition/ definierten Ausgangsposition befindet, so dass das Einführen des Entkopplungsinstruments in das Einsetzinstrument ohne ein vorheriges manuelles Drehen/ Ausrichten erfolgen kann.

Die Kulisse der Hülse erlaubt eine Verdrehung des Schafts in einem vordefinierten Winkel, bevorzugt um etwa 90° im Uhrzeigersinn, und kann mittels einer kleinen Vertiefung, die an einem Ende der Kulisse vorgesehen ist, ein taktiles Feedback für das Entkoppeln/ Spreizen des Einsetzinstruments bereitstellen. Diese Vertiefung ist aber für die Funktion der Kulisse nicht unbedingt notwendig.

Ein unbeabsichtigtes Herausrutschen des Entkopplungsinstruments aus dem Einsetzinstrument wird durch das Druckstück bzw. den Einrastmechanismus, die Vertiefung an der Kulisse, sowie den Kontakt zwischen dem Entkopplungsinstrument und dem Einsetzinstrument an dem distalen Ende verhindert.

Zum Entkoppeln der beiden Instrument (Entkopplungsinstrument und Einsetzinstrument) kann das Entkopplungsinstrument (der Schaft desselben), bevorzugt um 90° im Gegenuhrzeigersinn, (unterstützt durch die Federkraft) zurückgedreht werden, so dass sich die Hülse wieder in der Nullposition/ definierten Ausgangsposition für die nächste Anwendung befindet.

Es wird somit ein Entkopplungsinstrument bereitgestellt, welches eine definierte Nullposition und eine automatische Ausrichtung bereitstellt, welches keinen Formschluss zwischen sich und dem Implantat/ der Pedikelschraube erfordert, und welches bevorzugt ferner ein taktiles Feedback für das Entkoppeln liefert. Eine definierte Aufspreizung des Einsetzinstruments wird durch die Kulisse und die durch die (Druck-) Feder bewirkte Instrumentenausrichtung (Ausrichtung von Schaft und Hülse) sichergestellt.

### Kurzbeschreibung der Figuren

Die Erfindung wird nachfolgend mit Hilfe von Figuren weiter erläutert. Es zeigen:
- Fig. 1: eine Schnittansicht eines aus dem Stand der Technik bekannten Entkopplungsinstrument;
- Fig. 2: eine isometrische Detailansicht eines unteren Bereichs des Entkopplungsinstruments von Fig. 1;
- Fig. 3: eine isometrische Detailansicht eines oberen Bereichs des Entkopplungsinstruments von Fig. 1;
- Fig. 4: eine isometrische Ansicht des in ein Einsetzinstrument eingeführten Entkopplungsinstruments von Fig. 1;
- Fig. 5: eine isometrische Ansicht der erfindungsgemäßen medizintechnischen Anordnung umfassend das erfindungsgemäße Entkopplungsinstrument und ein Einsetzinstrument;
- Fig. 6: eine isometrische Ansicht des erfindungsgemäßen Entkopplungsinstrument;
- Fig. 7: eine erste isometrische Detailansicht des Entkopplungsinstruments von Fig. 5; und
- Fig. 8: eine zweite isometrische Detailansicht des Entkopplungsinstruments von Fig. 5.

### Detaillierte Beschreibung

Die Figuren sind lediglich schematischer Natur und dienen ausschließlich dem Verständnis der Erfindung. Gleiche Elemente sind mit denselben Bezugszeichen versehen.

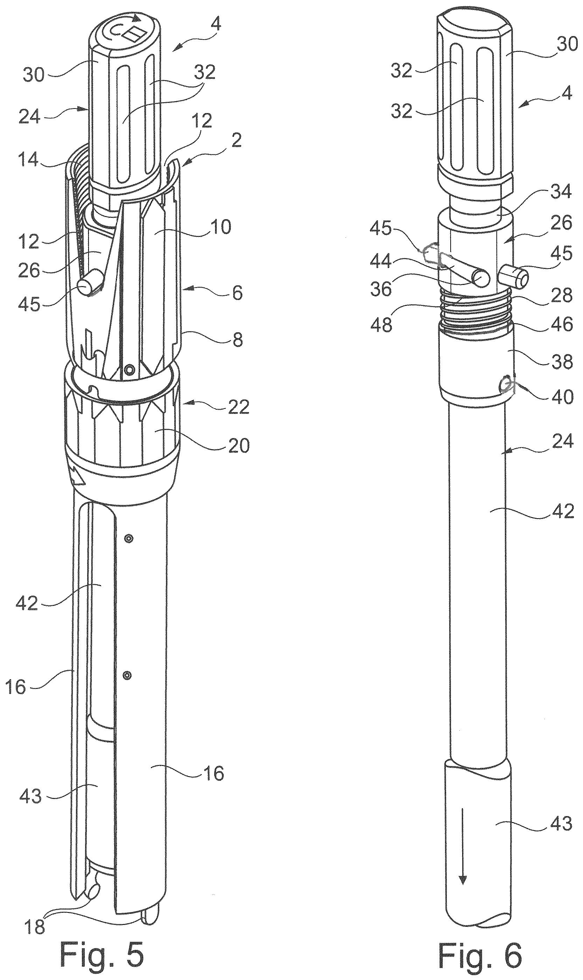

Fig. 5 zeigt eine medizintechnische Anordnung 2, welche (unter anderem) ein medizintechnisches Entkopplungsinstrument 4 und ein medizintechnisches Einsetzinstrument 6 umfasst.

Das Einsetzinstrument 6 weist einen Hauptkörper 8 auf. Dieser bildet einen oberen Aufnahmeabschnitt 10, unter anderem für das Entkopplungsinstrument 4, aus. Im Bereich des oberen Aufnahmeabschnitts 10 weist das Einsetzinstrument 6 zwei sich in Axialrichtung erstreckende V-förmige bzw. U-förmige Ausnehmungen 12 auf, welche zur Aufnahme von entsprechenden, an dem Entkopplungsinstrument 4 vorgesehenen Eingriffsstrukturen eingerichtet sind. Außerdem weist das Einsetzinstrument 6 im Bereich des oberen Aufnahmeabschnitts 10 einen inneren (Innen-)Gewindeabschnitt 14 auf, welcher zur Aufnahme, insbesondere zum Einschrauben, eines anderen, nicht dargestellten Instruments der medizintechnischen Anordnung 2 eingerichtet ist. Beispielsweise kann der innere Gewindeabschnitt 14 einen sogenannten Insert-Pusher bzw. Insert-Drücker aufnehmen, welcher einer Fixierung einer Polyaxialität bei einer polyaxialen Pedikelschraube (durch Drücken auf ein "Insert") dient.

An den oberen Aufnahmeabschnitt 10 des Hauptkörpers 8 schließen sich zwei einander diametral gegenüberliegende, sich parallel in Axialrichtung erstreckende Koppelarme 16 an. Die Koppelarme 16 weisen an ihren distalen Enden Raststrukturen, insbesondere Rastnasen 18 auf, welche grundsätzlich mit Gegenraststrukturen in Eingriff bringbar sind, welche an einer Aufnahmehülse/ Tulpe einer polyaxialen oder monoaxialen Pedikelschraube (nicht dargestellt) ausgebildet sind. Das Einsetzinstrument 6 weist weiterhin eine Verriegelungshülse 20 auf, welche in einem Übergangsbereich 22 zwischen dem oberen Aufnahmeabschnitt 10 und den Koppelarmen 16 um den Hauptkörper 8 herum angeordnet ist.

Das Einsetzinstrument 6 ist grundsätzlich eingerichtet, an seinen Rastnasen 18 eine Pedikelschraube, insbesondere eine Aufnahmehülse/ Tulpe der Pedikelschraube aufzunehmen. In einer ersten Freigabestellung der Verriegelungshülse 20 weisen die Koppelarme 16 eine geeignete Elastizität/ Biegsamkeit auf, um über ihre Rastnasen 18 die Pedikelschraube, insbesondere die Gegenraststrukturen der Pedikelschraube, aufzunehmen. Durch ein Drehen der Verriegelungshülse 20 von der ersten Freigabestellung in eine zweite Verriegelungsstellung wird der Hauptkörper 8 in Bezug auf die Verriegelungshülse 20 axial nach oben gezogen. Dadurch werden die Koppelarme 16 durch die Verriegelungshülse 20 fixiert, so dass die Pedikelschraube fest und sicher von dem Einsetzinstrument 6 gehalten wird.

Wenn ein Operateur/ Anwender nach erfolgter Verschraubung/ Verankerung der Pedikelschraube das Einsetzinstrument 6 von der Pedikelschraube entkoppeln möchte, muss dieser die Verriegelungshülse 20 wieder von der zweiten Verriegelungsstellung in die erste Freigabestellung verbringen (durch Verdrehen der Verriegelungshülse 20). Außerdem führt der Operateur/ Anwender das Entkopplungsinstrument 4 in das Einsetzinstrument 6 ein. Mit Hilfe des Entkopplungsinstruments können die Koppelarme 16 des Einsetzinstruments 6 gespreizt/ radial nach außen gedrückt werden, wodurch die Entkopplung der Pedikelschraube von dem Einsetzinstrument 6 bewirkt wird.

Das erfindungsgemäße medizintechnische Entkopplungsinstrument 4 ist in Fig. 6, Fig. 7 und Fig. 8 genauer dargestellt.

Das Entkopplungsinstrument 4 weist grundsätzlich einen Schaft/ Stab 24, eine Hülse 26 und eine Feder 28 auf.

Der Schaft 24 weist einen proximalen Griffabschnitt 30 an seinem proximalen Ende auf. Der proximale Griffabschnitt 30 ist im gezeigten bevorzugten Beispiel ovalzylindrisch ausgebildet (Zylinderform mit ovalem Querschnitt) und weist eine Vielzahl von sich in Axialrichtung erstreckende, in Umfangsrichtung gleichmäßig beabstandete/ verteilte längliche Ausnehmungen 32 auf, welche einem verbesserten Greifen des Schafts 24 durch einen Anwender/ Operateur dienen.

An den proximalen Griffabschnitt 30 schließt sich in Axialrichtung des Schafts 24 ein erster zylindrischer Abschnitt 34 mit rundem/ kreisförmigem Querschnitt an. Der erste zylindrische Abschnitt 34 weist einen sich radial nach außen erstreckenden zapfenförmigen Vorsprung 36 auf.

An den ersten zylindrischen Abschnitt 34 schließt sich in Axialrichtung des Schafts 24 ein zweiter zylindrischer Abschnitt 38 mit rundem/ kreisförmigem Querschnitt an. Der zweite zylindrische Abschnitt 38 weist einen größeren Durchmesser als der erste zylindrische Abschnitt 34 auf. An dem zweiten zylindrischen Abschnitt 38 ist ein Druckstück bzw. Einrastmechanismus 40 vorgesehen/ angeordnet. Das Druckstück 40 erzeugt eine Druckkraft radial nach außen und kann somit gegen das Einsetzinstrument 6 drücken, so dass ein unbeabsichtigtes Herausrutschen des Entkopplungsinstruments 4 verhindert wird. Ein unbeachsichtigtes Herausrutschen des Entkopplungsinstruments4 kann auch einen anderen Einrastmechanismus verhindert werden.

An den zweiten zylindrischen Abschnitt 38 schließt sich in Axialrichtung des Schafts 24 ein dritter zylindrischer Abschnitt 42 mit bevorzugt rundem/ kreisförmigen Querschnitt an. Der dritte zylindrische Abschnitt 42 weist einen gleichen oder kleineren Durchmesser als der zweite zylindrische Abschnitt 38 auf.

An den dritten zylindrischen Abschnitt 42 schließt sich in Axialrichtung des Schafts 24 ein distaler Eingriffsabschnitt 43 an, welcher das distale Ende des Schafts 24 bildet. Der distale Eingriffsabschnitt 43 ist zylindrisch mit unrundem Querschnitt geformt.

Die Hülse 26 des Entkopplungsinstruments 4 ist grundsätzlich hohlzylinderförmig ausgebildet und um den ersten zylindrischen Abschnitt 34 des Schafts 24 angeordnet. Die Hülse 26 weist eine Kulisse/ ein Langloch 44 auf, in welcher/ welchem der an dem Schaft 24 vorgesehene zapfenförmige Vorsprung 36 aufgenommen bzw. geführt ist. Die Kulisse 44 definiert einen Verdrehbereich des Schafts 24 in Bezug auf die Hülse 26. Insbesondere erlaubt die Kulisse 44 der Hülse 26 etwa eine 90°-Drehung des Schafts 24. Die Hülse 26 weist zwei zapfenförmige Vorsprünge 45 auf, welche einander diametral gegenüberliegen und sich radial nach außen erstrecken. Die zapfenförmigen Vorsprünge 45 der Hülse 26 sind vorgesehen, dass sie in den V-förmigen bzw. U-förmigen Ausnehmungen 12 des Einsetzinstruments 6 aufgenommen werden, so dass die Hülse 26 verdrehsicher in dem Einsetzinstrument 6 gehalten ist/ wird.

Die Feder 28 ist eine Spiralfeder bzw. eine Druckfeder und ist zwischen einer an einem proximalen Ende des zweiten zylindrischen Abschnitts 38 des Schafts 24 ausgebildeten Auflagefläche 46 (für die Feder 28) und einer an einem distalen Ende der Hülse 26 ausgebildeten Anlagefläche 48 (für die Feder 28) angeordnet.

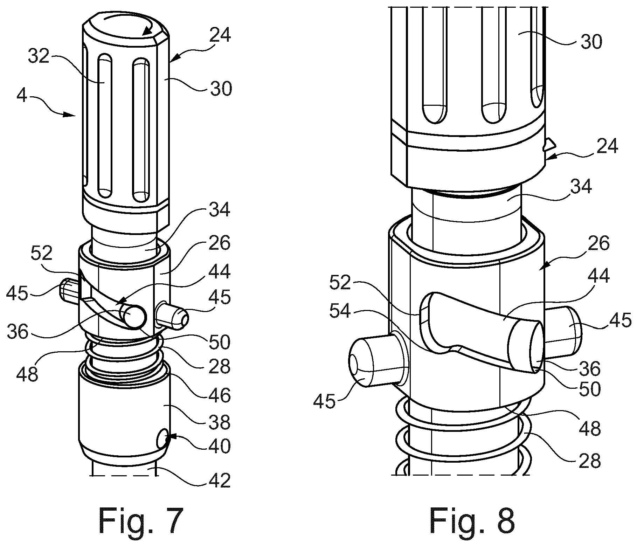

Die Kulisse 44 der Hülse 26 verläuft schräg, das heißt erstreckt sich sowohl in Umfangsrichtung als auch in Axialrichtung der Hülse 26. Das bedeutet, dass ein erstes Ende 50 der Kulisse 44 näher an einem distalen Ende der Hülse 26 angeordnet ist als ein zweites Ende 52 der Kulisse 44. Die besonders aus Fig. 7 oder Fig. 8 hervorgehende Form der Kulisse 44 kann auch gespiegelt vorliegen/ ausgebildet sein (Ausführungsform für Linkshänder).

Die Feder 28 bringt eine Druckkraft auf die Hülse 26 auf und drückt die Hülse 26 axial nach oben. Dadurch wird der zapfenförmige Vorsprung 36 des Schafts 24 automatisch bzw. selbsttätig gegen das erste Ende 50 der Kulisse 44 gedrückt. Wenn sich der zapfenförmige Vorsprung 36 des Schafts 24 an dem ersten Ende 50 der Kulisse/ des Langlochs 44 befindet, stellt dies eine definierte Ausgangsposition/ Nullposition/ ursprüngliche Position dar. In dieser definierten Ausgangsposition sind die Hülse 26 und der Schaft 24 derart zueinander angeordnet, dass, wenn die zapfenartigen Vorsprünge 45 der Hülse 26 in den V-förmigen Ausnehmungen 12 des Einsetzinstruments 6 aufgenommen und verdrehsicher gehalten sind, der unrunde distale Eingriffsabschnitt 43 des Schafts 24 bezüglich der Koppelarme 16 des Einsetzinstruments 6 derart angeordnet ist, dass die Koppelarme 16 nicht gespreizt werden. In anderen Worten sind der Schaft/ Stab 24 und die Hülse 26 des Entkopplungsinstruments 4 automatisch zueinander für das Einsetzen/ Einführen in das Einsetzinstrument 6 ausgerichtet.

Wenn das Entkopplungsinstrument 4 in das Einsetzinstrument 6 eingesetzt ist, wird die Hülse 26 (die zapfenförmigen Vorsprünge 45 der Hülse 26) verdrehsicher in den V-förmigen bzw. U-förmigen Ausnehmungen 12 des Einsetzinstruments 6 gehalten. Ein Anwender/ Operateur kann nun den Schaft 24 bezüglich der Hülse 26 durch ein Drehen des proximalen Griffabschnitts 30 im Uhrzeigersinn um etwa 90° verdrehen, bis sich der zapfenförmige Vorsprung 36 des Schafts 24 an dem zweiten Ende 52 der Kulisse/ des Langlochs 44 befindet. In dieser Position spreizt der ovalförmige distale Eingriffsabschnitt 43 des Schafts 24 die Koppelarme 16 des Einsetzinstruments 6 auf, so dass eine Entkopplung des Einsetzinstruments 6 von der Pedikelschraube erfolgt.

Wie insbesondere aus Fig. 8 hervorgeht, kann die Kulisse/ das Langloch 44 an seinem zweiten Ende 52 eine Vertiefung/ Einkerbung/ Mulde 54 aufweisen. Es ist jedoch auch denkbar, dass die Vertiefung 54 nicht vorgesehen ist. Wenn der zapfenförmige Vorsprung 36 des Schafts 24 an dem zweiten Ende 52 der Kulisse 44 in die Vertiefung 54 rutscht bzw. fällt bzw. aufgenommen wird, das heißt insbesondere durch die Feder 28 in die Vertiefung 54 gedrückt wird, wird einem Anwender/ Operateur ein taktiles Feedback gegeben, welches dem Anwender/ Operateur anzeigt, dass das Einsetzinstrument 6 von der Pedikelschraube entkoppelt ist.

Ein unbeabsichtigtes Herausrutschen des Entkopplungsinstruments 4 wird in dieser Position durch das Druckstück 40, welches von innen gegen das Einsetzinstrument 6 drückt, die Vertiefung 54 an der Kulisse 44, sowie den Kontakt zwischen dem Entkopplungsinstrument 4 und dem Einsetzinstrument 6 an deren distalem Ende verhindert.

Zum Entkoppeln der beiden Instrumente (Entkopplungsinstrument 4 und Einsetzinstrument 6) kann das Entkopplungsinstrument 4 um 90° im Gegenuhrzeigersinn (unterstützt durch die Federkraft) zurückgedreht werden, so dass sich die Hülse 26 wieder in der Nullposition/ Ausgangsposition für die nächste Anwendung befindet.

### Bezugszeichenliste

- 2: medizintechnische Anordnung
- 4: Entkopplungsinstrument
- 6: Einsetzinstrument
- 8: Hauptkörper
- 10: oberer Aufnahmeabschnitt
- 12: V-förmige Ausnehmung
- 14: innerer Gewindeabschnitt
- 16: Koppelarm
- 18: Rastnase
- 20: Verriegelungshülse
- 22: Übergangsbereich
- 24: Schaft/ Stab
- 26: Hülse
- 28: Feder
- 30: proximaler Griffabschnitt
- 32: längliche Ausnehmung
- 34: erster zylindrischer Abschnitt
- 36: zapfenförmiger Vorsprung (des Schaft/ Stabs)
- 38: zweiter zylindrischer Abschnitt
- 40: Druckstück
- 42: dritter zylindrischer Abschnitt
- 43: distaler Eingriffsabschnitt
- 44: Kulisse/ Langloch
- 45: zapfenförmiger Vorsprung (der Hülse)
- 46: Auflagefläche
- 48: Anlagefläche
- 50: erstes Ende
- 52: zweites Ende
- 54: Vertiefung/ Einkerbung/ Mulde

## Patentansprüche

1. Medizintechnisches Entkopplungsinstrument (4) zum Entkoppeln eines medizintechnischen Einsetzinstruments (6) von einer Pedikelschraube, mit: einem Schaft (24) und einer um einen Abschnitt (34) des Schafts (24) angeordneten Hülse (26), welche zur definierten Ankopplung an das medizintechnische Einsetzinstrument (6) eingerichtet ist, wobei der Schaft (24) bezüglich der Hülse (26) verdrehbar ist, und wobei das medizintechnische Entkopplungsinstrument (4) eingerichtet ist, den Schaft (24) und die Hülse (26) selbsttätig und automatisch in eine definierte Ausgangsposition zu verbringen; wobei das medizintechnisches Entkopplungsinstrument (4) ferner eine Feder (28) aufweist, welche zwischen einer an dem Schaft (24) vorgesehenen Auflagefläche (46) und der Hülse (26) angeordnet ist und eingerichtet ist, über ihre Federkraft den Schaft (24) und die Hülse (26) in die definierte Ausgangsposition zu verbringen; wobei der Schaft (24) an dem Abschnitt (34), um welchen die Hülse (26) angeordnet ist, einen zapfenförmigen Vorsprung (36) aufweist, die Hülse (26) eine Kulisse (44) aufweist, und der zapfenförmige Vorsprung (36) des Schafts (24) in der Kulisse (44) der Hülse (26) aufgenommen ist, und zwar derart, dass die Kulisse (44) einen Verdrehbereich des Schafts (24) in Bezug auf die Hülse (26) definiert;
**dadurch gekennzeichnet, dass**
die Kulisse (44) schräg verläuft, sich also sowohl in Umfangsrichtung als auch in Axialrichtung der Hülse (26) erstreckt, so dass ein erstes Ende (50) der Kulisse (44) näher an einem distalen Ende der Hülse (26) angeordnet ist als ein zweites Ende (52) der Kulisse (44).

2. Medizintechnisches Entkopplungsinstrument (4) nach Anspruch 1, wobei in der definierten Ausgangsposition der zapfenförmige Vorsprung (36) des Schafts (24) gegen das erste Ende (50) der Kulisse (44) durch die Feder (28) gedrückt ist.

3. Medizintechnisches Entkopplungsinstrument (4) nach Anspruch 1 oder 2, wobei die Kulisse (44) an ihrem zweiten Ende (52) eine Vertiefung (54) aufweist, so dass der zapfenförmige Vorsprung (36) des Schafts (24) mittels der Feder (28) in die Vertiefung (54) gedrückt werden kann.

4. Medizintechnisches Entkopplungsinstrument (4) nach einem der vorhergehenden Ansprüche, wobei die Hülse (26) zur definierten Ankopplung an das medizintechnische Einsetzinstrument (6) zapfenförmige Vorsprünge (45) aufweist.

5. Medizintechnisches Entkopplungsinstrument (4) nach einem der vorhergehenden Ansprüche, wobei der Schaft (24) einen distalen Eingriffsabschnitt (43) aufweist, welcher zylindrisch mit unrundem Querschnitt geformt ist.

6. Medizintechnisches Entkopplungsinstrument (4) nach einem der vorhergehenden Ansprüche, wobei ein Abschnitt (38) des Schafts (24) ein Druckstück bzw. einen Einrastmechanismus (40) aufweist, welches eingerichtet ist, eine Druckkraft radial nach außen zu erzeugen bzw. aufzubringen.

7. Medizintechnische Anordnung (2) umfassend zumindest das medizintechnische Entkopplungsinstrument (4) nach einem der vorhergehenden Ansprüche und ein medizintechnisches Einsetzinstrument (6).

## Claims

1. A medical decoupling instrument (4) for decoupling a medical insertion instrument (6) from a pedicle screw, comprising: a shaft (24) and a sleeve (26) which is arranged around a portion (34) of the shaft (24), said sleeve (26) being configured for defined coupling to the medical insertion instrument (6), wherein the shaft (24) is rotatable with respect to the sleeve (26), and wherein the medical decoupling instrument (4) is configured to move the shaft (24) and the sleeve (26) self-actingly and automatically into a defined initial position; wherein the medical decoupling instrument (4) further comprises a spring (28) which is arranged between a support surface (46), which is provided on the shaft (24), and the sleeve (26) and is configured to move the shaft (24) and the sleeve (26) into the defined initial position via its spring force; wherein the shaft (24) has a peg-shaped projection (36) at the portion (34) around which the sleeve (26) is arranged, the sleeve (26) has a motion link (44), and the peg-shaped projection (36) of the shaft (24) is received in the motion link (44) of the sleeve (26) such that the motion link (44) defines a range of rotation of the shaft (24) with respect to the sleeve (26); **characterized in that** the motion link (44) runs obliquely, i.e. extends both in the circumferential direction and the axial direction of the sleeve (26), such that a first end (50) of the motion link (44) is arranged closer to a distal end of the sleeve (26) than a second end (52) of the motion link (44).

2. The medical decoupling instrument (4) according to claim 1, wherein, in the defined initial position, the peg-shaped projection (36) of the shaft (24) is pressed against the first end (50) of the motion link (44) by the spring (28).

3. The medical decoupling instrument (4) according to claim 1 or 2, wherein the motion link (44) has a depression (54) at its second end (52) such that the peg-shaped projection (36) of the shaft (24) can be pressed into the depression (54) via the spring (28).

4. The medical decoupling instrument (4) according to one of the preceding claims, wherein the sleeve (26) has peg-shaped projections (45) for defined coupling to

5. The medical decoupling instrument (4) according to one of the preceding claims, wherein the shaft (24) has a distal engagement portion (43) that is cylindrically shaped with a non-circular cross-section.

6. The medical decoupling instrument (4) according to one of the preceding claims, wherein a portion (38) of the shaft (24) has a push piece or an engagement mechanism (40) that is configured to apply a push force radially outward.

7. A medical assembly (2) comprising at least the medical decoupling instrument (4) according to one of the preceding claims and a medical insertion instrument (6).

## Revendications

1. Instrument de découplage (4) à usage médical pour le découplage d'un instrument d'insertion (6) à usage médical d'une vis pédiculaire, avec : une tige (24) et un manchon (26) agencé autour d'une section (34) de la tige (24), manchon qui est conçu pour le couplage défini à l'instrument d'insertion (6) à usage médical, dans lequel la tige (24) est rotative par rapport au manchon (26), et dans lequel l'instrument de découplage (4) à usage médical est conçu afin d'amener la tige (24) et le manchon (26) de manière autonome et automatiquement dans une position initiale définie ; dans lequel l'instrument de découplage (4) à usage médical présente de plus un ressort (28) qui est agencé et conçu entre une surface d'appui (46) prévue au niveau de la tige (24) et le manchon (26) afin d'amener par le biais de la force de ressort la tige (24) et le manchon (26) dans la position initiale définie ; dans lequel la tige (24) présente au niveau de la section (34), autour de laquelle le manchon (26) est agencé, une saillie (36) en forme de tenon, le manchon (26) présente une coulisse (44), et la saillie (36) en forme de tenon de la tige (24) est logée dans la coulisse (44) du manchon (26), et ce de telle manière que la coulisse (44) définisse une zone de rotation de la tige (24) par rapport au manchon (26) ; **caractérisé en ce que**
la coulisse (44) s'étend en biais, passe donc non seulement dans le sens périphérique mais aussi dans le sens axial du manchon (26) de sorte qu'une première extrémité (50) de la coulisse (44) soit agencée plus près d'une extrémité distale du manchon (26) qu'une seconde extrémité (52) de la coulisse (44).

2. Instrument de découplage (4) à usage médical selon la revendication 1, dans lequel dans la position initiale définie, la saillie (36) en forme de tenon de la tige (24) est pressée contre la première extrémité (50) de la coulisse (44) par le ressort (28).

3. Instrument de découplage (4) à usage médical selon la revendication 1 ou 2, dans lequel la coulisse (44) présente à sa seconde extrémité (52) une cavité (54) de sorte que la saillie (36) en forme de tenon de la tige (24) puisse être pressée au moyen du ressort (28) dans la cavité (54).

4. Instrument de découplage (4) à usage médical selon l'une quelconque des
revendications précédentes, dans lequel le manchon (26) présente des saillies (45) en forme de tenon pour le couplage défini à l'instrument d'insertion (6) à usage médical.

5. Instrument de découplage (4) à usage médical selon l'une quelconque des
revendications précédentes, dans lequel la tige (24) présente une section de prise (43) distale qui est formée de manière cylindrique avec une section transversale non ronde.

6. Instrument de découplage (4) à usage médical selon l'une quelconque des
revendications précédentes, dans lequel une section (38) de la tige (24) présente une pièce de pression ou un mécanisme d'encliquetage (40) qui est conçu(e) afin de générer ou d'appliquer une force de pression radialement vers l'extérieur.

7. Ensemble (2) à usage médical comprenant au moins
l'instrument de découplage (4) à usage médical selon l'une quelconque des revendications précédentes et un instrument d'insertion (6) à usage médical.
